# EUROPEAN PATENT APPLICATION

(11) **EP 1 621 637 A1**
(43) Date of publication of application: **01.02.2006**
(21) Application number: 04291959.7
(22) Date of filing: 30.07.2004
(51) Int. Cl.: C12Q 1/48, G01N 33/574, C07K 16/00

(54) **ppGalNac-T6 mRNA or peptide as a new marker for the detection of cancer cells**

(71) Applicant: Institut Curie, 75005 Paris (FR)
(72) Inventor: Osinaga, Eduardo, 11400 Montevideo (UY); Freire, Teresa, 94200 Ivry Sur Seine (FR); Berois, Nora, 11400 Montevideo (UY); Sastre-Garau, Xavier, 94000 Vincennes (FR); Magdelenat, Henri, 75007 Paris (FR)
(74) Representative: Texier, Christian

(57) **Abstract**

The invention is directed to UDP-*N*-Acetyl-D-galactosamine: polypeptide *N*-Acetylgalactosaminyltransferase 6 (ppGalNAc-T6) mRNA or peptide as a new marker for method for diagnosing or prognosticating cancer in a subject, particularly for measuring the aggressiveness of cancer in a subject or for determining the presence of disseminated cancer cells in a subject with cancer. The invention further comprises specific anti-human ppGalNAc-T6 polyclonal or monoclonal antibodies, hybridoma cells capable of secreting such monoclonal antibodies and kits comprising such antibodies. The present invention also pertains to a method for the selection of anticancer compounds capable of inhibiting the ppGalNAc-T6 mRNA or peptide in cell.

## Description

The present invention is directed to UDP-*N*-Acetyl-D-galactosamine: polypeptide *N*-Acetylgalactosaminyltransferase 6 (ppGalNAc-T6) mRNA or peptide as a new marker for method for diagnosing or prognosticating cancer in a subject, particularly for measuring the aggressiveness of cancer in a subject or for determining the presence of disseminated cancer cells in a subject with cancer. The invention further comprises specific anti-human ppGalNAc-T6 polyclonal or monoclonal antibodies, hybridoma cells capable of secreting such monoclonal antibodies and kits comprising such antibodies. The present invention also pertains to a method for the selection of anti-cancer compounds capable of inhibiting the ppGalNAc-T6 mRNA or peptide in cell.

The increased number of cancer cases reported around the world, is a major concern. Currently there are only a handful of treatments available for specific types of cancer, and these treatments require not only an early detection of the malignancy, but also a reliable assessment of the severity of the malignancy.

Breast cancer is the leading cause of malignancies among women worldwide (Parkin et al., Int. J. Cancer, 80:827-841, 1999). Thanks to the usage of radiological screening tests and to an increasing awareness among women, more breast cancer patients are diagnosed in an early, node-negative stage (Feig, Cancer, 80:2035-2039, 1997). These patients, without evidence of distant metastases at time of presentation, had been thought to require no adjuvant treatment. However, clinical evidence shows that approximately 30 % of node-negative breast cancer patients will later present with distant disease and will subsequently die from their malignant disease. This observation might be explained by an occult dissemination of tumour cells that is already present at the time of primary diagnosis but is not detected by conventional tumour staging. There is now increasing evidence that the detection of disseminated malignant cells in bone marrow aspirates of patients with early stages of breast cancer is of prognostic relevance (Braun et al., N. Engl. J. Med., 342:525-533, 2000; Gerber et al., J. Clin. Oncol., 19:969-971, 2001), since abundant clinical and experimental data suggest that these cells are precursors of subsequent distant relapse (Riethmuller and Klein, Semin. Cancer Biol., 11:307-311, 2001; Schmidt-Kittler et al., Proc. Natl. Acad. Sci. (USA), 100:7737-7742, 2003). Therefore, the identification of occult metastases could influence future strategies of adjuvant systemic therapy (Braun et al., J. Clin. Oncol., 19:1468-1475, 2001; Syme et al., Bone Marrow Transplant., 32:307-311, 2003) .Several molecular methods have been employed for searching epithelial cell-specific protein expression in nonepithelial tissues. The application of monoclonal antibodies directed against surface antigens or cytokeratins has led to improvements in the ability to detect breast cancer cells by different immunolabelling techniques (Pantel and von Knebel Doeberitz, Curr. Opin. Oncol., 12:95-101, 2000; von Knebel Doeberitz et al., Recent Results Cancer Res., 158:181-186, 2001). The use of RT-PCR has been a highly sensitive tool in the detection of isolated breast cancer cells amplifying several transcripts, for example CK19 (Stathopoulou et al., J. Clin. Oncol., 20:3404-3412, 2002), CEA (Zhong et al., J. Cancer. Res Clin. Oncol., 125:669-674, 1999), mammaglobin (Silva et al., Ann Oncol., 13: 422-429, 2002) maspin (Luppi et al., Ann Oncol., 7:619-624, 1996) and MUC5B (Berois et al., Int. J. Cancer, 103:550-555, 2003). However, despite numerous publications in this field, it has not been possible to establish the ideal method (Lugo et al., J. Clin. Oncol., 21:2609-2615, 2003).

Glycosyltransferases constitute a large group of enzymes that are involved in the biosynthesis of oligosaccharides and polysaccharides (Breton et al., Biochimie, 83:713-718, 2001). It is well known that tumour development is usually associated with alterations in cell-surface carbohydrates. Glycosylation changes arising as a consequence of malignant transformation influence cell growth as well as the differentiation, adhesiveness and immunogenicity of cancer cells (Fukuda, Cancer Res., 56:2237-2244, 1996; Hollingsworth and Swanson, Nat Rev. Cancer., 4:45-60, 2004). Moreover, it has been shown that carbohydrate structures on the tumour cell surface are associated with the metastatic potential of tumour cells, and even the prognosis of cancer patients (Gorelik et al.; Cancer Metastasis Rev., 20:245-277, 2001). One of the reasons why these tumour-associated carbohydrate antigens are produced is a deregulation of glycosyltransferases, resulting in changes in enzyme activity and/or specificity for specific substrates. It has been shown that several glycosyltransferases are useful tumour markers, for example, core 2 β1,6-N-acetylglucosaminyltransferase mRNA is a good marker for lung carcinomas (Machida et al., Cancer Res., 61:2226-2231, 2001), and expression of pl,6-N-acetylglucosaminyltransferase V (GnTV) (Murata et al., Clin. Cancer Res., 6:1772-1777, 2000) and sialyltransferase CMP-sialic acid: Galβ1,3GaINAc-R α6-sialyltransferase (Schneider et al., Cancer Res., 61:4605-4611, 2001) correlates with a poor prognosis and low patient survival in human colorectal carcinomas. Recently it was reported that the detection of mRNA of α,1,4-N-acetylglucosaminyltransferase (Shimizu et al., Lab. Invest., 83:187-197, 2003) and the ganglioside GD2 synthetase (β1,4-N-acetylgalactosaminyltransferase) (Cheung et al., J. Clin. Oncol., 21:1087-1093, 2003) are useful procedures to detect circulating cells from gastric cancer and neuroblastoma, respectively.

An incomplete elongation of *O*-glycan saccharide chains in mucins has been associated with malignant breast transformation (Burchell et al., J. Mammary Gland Biol. Neoplasia, 6:355-364, 2001). Mucin-type linkages (GaINAc-O-Ser/Thr) are initiated by a family of glycosyltransferases called UDP-*N*-Acetyl-D-galactosamine: Polypeptide *N*-Acetylgalactosaminyltransferases (ppGalNAc-Ts, EC 2.4.2.41) (Ten Hagen et al., Glycobiology, 13:1R-16R, 2003).

Currently there is a need for new methods in the fight against cancer, particularly against breast cancer and it would therefore be beneficial to provide specific methods and reagents for the diagnosis, staging, prognosis, and monitoring of cancer.

This is the object of the present invention.

Considering that ppGalNAc-Ts isoforms display tissue-specific expression in adult mammals, the inventors have performed a study in order to evaluate the potential utility of ppGalNAc-T mRNA expression to detect disseminated human cancer cells, notably in biological samples from breast cancer patients, particularly bone marrow samples.

Surprisingly, the inventors have demonstrated that ppGalNAc-T6 gene expression is a marker for tumor cells, particularly for metastasic cancer and disseminated cancer cells.

So, the present invention provides an *in vitro* method for diagnosing or prognosticating cancer in a subject, this method comprises the step of determining the presence or the absence of an expression product of the gene encoding ppGalNAc-T6 in a biological sample from the subject wherein the presence of ppGalNAc-T6 expression product is indicative of cancer.

In another aspect, the invention provides an *in vitro* method for measuring the aggressiveness of cancer in a subject, this method comprises the step of determining the presence or the absence of the expression product of the gene encoding ppGalNAc-T6 in a biological sample from the subject wherein the presence of ppGalNAc-T6 expression product is indicative of the aggressiveness of the cancer.

In a further aspect, the invention is directed to an *in vitro* method for determining the presence of disseminated cancer cells in a biological sample of a cancer subject, wherein said disseminated cancer cells are identifying by the presence of an expression product of the gene encoding ppGalNAc-T6 and wherein the presence of ppGalNAc-T6 expression product in cells is indicative of the presence of disseminated cancer cells.

In another embodiment, the invention encompasses an *in vitro* method for identifying metastatic cancer in a biological sample from a subject with cancer or for determining if a subject is at risk of developing metastatic, wherein said method comprises the step of determining the presence of disseminated cancer cells in such biological sample according to the method for determining the presence of disseminated cancer cells of the present invention and wherein the presence of disseminated cancer cells is indicative of metastatic cancer or is indicative of a cancer that will become metastatic.

The present invention also comprises an *in vitro* method for determining the subject's response to an anti-cancer therapy, who is receiving or has received therapy for a state associated with cancer wherein said method comprises the step of determining the presence or the absence of an expression product of the gene encoding ppGalNAc-T6 in a biological sample from the subject and the presence of the expression product of the gene encoding ppGalNAc-T6 is indicative of the subject's response to the therapy.

The above method of the invention can be used in combination with other methods of cancer diagnosis or prognosis.

"Cancer" includes a malignant neoplasm characterized by deregulated or uncontrolled cell growth. The term "cancer" includes primary malignant tumors (e.g., those whose cells have not migrated to sites in the subject's body other than the site of the original tumor) and secondary malignant tumors (e. g., those arising from metastasis, the migration of tumor cells to secondary sites that are different from the site of the original tumor).

The term "aggressive" (or "invasive") as used herein with respect to cancer refers to the proclivity of a tumor for expanding beyond its boundaries into adjacent tissue, or to the characteristic of the tumor with respect to metastasis. Invasive cancer can be contrasted with organ-confined cancer. For example, a basal cell carcinoma of the skin is a non-invasive or minimally invasive tumor, confined to the site of the primary tumor and expanding in size, but not metastasizing. In contrast, the cancer melanoma is highly invasive of adjacent and distal tissues. The invasive property of a tumor is often accompanied by the elaboration of proteolytic enzymes, such as collagenases, that degrade matrix material and basement membrane material to enable the tumor to expand beyond the confines of the capsule, and beyond confines of the particular tissue in which that tumor is located.

The term "metastasis" or "metastatic" as used herein refers to the condition of spread of cancer from the organ of origin to additional distal sites in the patient. The process of tumor metastasis is a multistage event involving local invasion and destruction of intercellular matrix, intravasation into blood vessels, lymphatics or other channels of transport, survival in the circulation, extravasation out of the vessels in the secondary site and growth in the new location.

The term "subject" includes mammals, e. g., humans, dogs, cows, horses, kangaroos, pigs, sheep, goats, cats, mice, rabbits, rats, and transgenic non-human animals.

In the methods of the present invention, such cancer is preferably selected from the group consisting of breast cancer, ovarian cancer, digestive cancers and throat cancer is preferred, particularly of human subject, the more preferred is breast cancer.

The term "biological samples" includes solid and body fluid samples. The biological samples of the present invention may include cells, protein, blood or biological fluids such as bone marrow, ascites fluid or brain fluid (e. g., cerebrospinal fluid). Examples of solid biological samples include samples taken from feces, the rectum, central nervous system, bone, breast tissue, renal tissue, the uterine cervix, the endometrium, the head/neck, the gallbladder, parotid tissue, the prostate, the brain, the pituitary gland, kidney tissue, muscle, the esophagus, the stomach, the small intestine, the colon, the liver, the spleen, the pancreas, thyroid tissue, heart tissue, lung tissue, the bladder, adipose tissue, lymph node tissue, the uterus, ovarian tissue, adrenal tissue, testis tissue, the tonsils, and the thymus. Examples of "body fluid samples "include samples taken from the blood, serum, semen, prostate fluid, seminal fluid, urine, saliva, sputum, mucus, bone marrow, lymph, and tears.

Samples for use in the assays of the invention can be obtained by standard methods including venous puncture and surgical biopsy. In one embodiment, the biological sample is a breast tissue sample obtained by needle biopsy.

In a preferred embodiment, the biological sample used in the methods of the present invention is selected from the group consisting of bone marrow, serum, plasma, blood, lymph, or cells from the cancerous or suspected cancerous tissue or adjacent tissue thereof, preferably a bone marrow sample.

As described in more detail below, the detection methods of the invention can be used to detect ppGalNAc-T6 mRNA, ppGalNAc-T6 cDNA, ppGalNAc-T6 peptide, RT-PCR and quantitative RT-PCR or specific fragments thereof, in a biological sample *in vitro.* For example, *in vitro* techniques for detection of ppGalNAc-T6 mRNA include Northern hybridizations and in situ hybridizations. *In vitro* techniques for detection of ppGalNAc-T6 peptide include immunohistochemistry, enzyme linked immunosorbent assays (ELISAs), Western blots, immunoprecipitations, and immunofluorescence.

In a preferred embodiment, the invention relates to the methods according to the present invention, wherein the expression product of the ppGalNAc-T6 gene is ppGalNAc-T6 RNA, preferably mRNA. Human ppGalNAc-T6 mRNA having the sequence SEQ ID NO: 1 is the most preferred, or natural polymorphic variant thereof.

When the methods according to the present invention are based to the detection or the quantification of ppGalNAc-T6 mRNA expression product, it is also preferred that the determination of the presence, or the level, or the absence of ppGalNAc-T6 mRNA comprises a step of amplifying ppGalNAc-T6 mRNA or cDNA, the complementary sequence thereof, or a fragment thereof having at least 30 nucleotides length which is specific of ppGalNAc-T6, preferably such ppGalNAc-T6 mRNA having the sequence SEQ ID NO: 1.

As used herein, the term "cDNA" shall refer to the DNA copy of the mRNA transcript of a gene.

It is also preferred that the step of amplifying ppGalNAc-T6 mRNA or cDNA is performed by PCR or RT-PCR reaction.

This detection may be accomplished by isolating mRNA from a sample When using mRNA detection, the method may be carried out by converting the isolated mRNA to cDNA according to standard methods; treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of ppGalNAc-T6 specific nucleic acid in the biological sample.

In a more preferred embodiment, the invention pertains to the methods according to the present invention, wherein the pair of primers used for the PCR amplification is capable of amplifying a fragment comprising the sequence SEQ ID NO: 3 (nt 212-711 of the cDNA having the sequence SEQ ID NO: 1 encoding ppGalNAc-T6), or a fragment thereof having at least 20, 25, 30, 35, 40, 45, 50, 60, 75, 100 or more nucleotides length which is specific of human ppGalNAc-T6 mRNA.

A pair of primers having the sequence SEQ ID NO: 5 and SEQ ID NO: 6 also forms part of the present invention and are preferred for amplifying reaction.

The term "primer", as used herein, refers to an oligonucleotide, whether occurring naturally (as in a purified restriction digest) or produced synthetically, and which is capable of initiating synthesis of a strand complementary to a nucleic acid when placed under appropriate conditions, i.e., in the presence of nucleotides and an inducing agent, such as a DNA polyrnerase, and at a suitable temperature and pH. The primer may be either single-stranded or double-stranded and must be sufficiently long to prime the synthesis of the desired extension product in the presence of the inducing agent. The exact length of the primer will depend upon many factors, including temperature, sequence and/or homology of primer and the method used. For example, in diagnostic applications, the oligonucleotide primer typically contains 10 to 25 or more nucleotides, depending upon the complexity of the target sequence, although it may contain fewer nucleotides.

The primers herein are selected to be "substantially" complementary to particular target DNA sequences. This means that the primers must be sufficiently complementary to hybridize with their respective strands. Therefore, the primer sequence need not reflect the exact sequence of the template. For example, a non-complementary nucleotide fragment (i.e., containing a restriction site) may be attached to the 5' end of the primer, with the remainder of the primer sequence being complementary to the strand. Alternatively, non-complementary bases or longer sequences can be interspersed into the primer, provided that the primer sequence has sufficient complementary with the sequence to hybridize therewith and form the template for synthesis of the extension product.

"Amplifying" refers to template-dependent processes and vector-mediated propagation which result in an increase in the concentration of a specific nucleic acid molecule relative to its initial concentration, or in an increase in the concentration of a detectable signal. As used herein, the term template-dependent process is intended to refer to a process that involves the template-dependent extension of a primer molecule.

The term template dependent process refers to nucleic acid synthesis of an RNA or a DNA molecule wherein the sequence of the newly synthesized strand of nucleic acid is dictated by the well-known rules of complementary base pairing (see, for example, Watson, J. D. et al., In: Molecular Biology of the Gene, 4th Ed., W. A. Benjamin, Inc., Menlo Park, Calif. (1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by Maniatis T. et al., Molecular Cloning (A Laboratory Manual), Cold Spring Harbor Laboratory, 1982.

A number of template dependent processes are available to amplify the target sequences of interest present in a sample. One of the best known amplification methods is the polymerase chain reaction (PCR) which is described in detail in Mullis et al., US Patent No. 4,683,195, Mullis et al., US Patent No. 4,683,202, and Mullis et al., US Patent No. 4,800,159, and in Innis et al., PCR Protocols, Academic Press, Inc., San Diego Calif., 1990. Briefly, in PCR, two primer sequences are prepared which are complementary to regions on opposite complementary strands of the target sequence. An excess of deoxynucleoside triphosphates are added to a reaction mixture along with a DNA polymerase (e. g., Taq polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction products and the process is repeated. Preferably a reverse transcriptase PCR amplification procedure may be performed in order to quantify the amount of mRNA amplified. Polymerase chain reaction methodologies are well known in the art.

Another method for amplification is the ligase chain reaction (LCR), disclosed in European Patent No. EP 0 320 308. In LCR, two complementary probe pairs are prepared, and in the presence of the target sequence, each pair will bind to opposite complementary strands of the target such that they abut. In the presence of a ligase, the two probe pairs will link to form a single unit. By temperature cycling, as in PCR, bound ligated units dissociate from the target and then serve as "target sequences" for ligation of excess probe pairs. Whiteley et al., US Patent No. 4,883,750 describes an alternative method of amplification similar to LCR for binding probe pairs to a target sequence.

Qbeta Replicase, described in PCT Application No. PCT/US 87/00880 may also be used as still another amplification method in the present invention. In this method, a replicative sequence of RNA which has a region complementary to that of a target is added to a sample in the presence of an RNA polymerase. The polymerase will copy the replicative sequence which can then be detected.

Strand Displacement Amplification (SDA) is another method of carrying out isothermal amplification of nucleic acids which involves multiple rounds of strand displacement and synthesis, i.e. nick translation. A similar method, called Repair Chain Reaction (RCR) is another method of amplification which may be useful in the present invention and is involves annealing several probes throughout a region targeted for amplification, followed by a repair reaction in which only two of the four bases are present. The other two bases can be added as biotinylated derivatives for easy detection.

ppGalNAc-T6 mRNA specific sequences can also be detected using a cyclic probe reaction (CPR). In CPR, a probe having a 3'and 5'sequences of non-tumor specific DNA and middle sequence of tumor specific RNA is hybridized to DNA which is present in a sample. Upon hybridization, the reaction is treated with RNaseH, and the products of the probe identified as distinctive products generating a signal which are released after digestion. The original template is annealed to another cycling probe and the reaction is repeated. Thus, CPR involves amplifying a signal generated by hybridization of a probe to a tumor cancer specific expressed nucleic acid.

Still other amplification methods described in GB Application No. 2 202 328, and in PCT Application No. PCT/US 89/01025 may be used in accordance with the present invention. In the former application, "modified" primers are used in a PCR like, template and enzyme dependent synthesis. The primers may be modified by labelling with a capture moiety (e. g., biotin) and/or a detector moiety (e. g., enzyme). In the latter application, an excess of labelled probes are added to a sample. In the presence of the target sequence, the probe binds and is cleaved catalytically. After cleavage, the target sequence is released intact to be bound by excess probe. Cleavage of the labelled probe-signals the presence of the target sequence.

Other nucleic acid amplification procedures include transcription-based amplification systems (TAS) (Kwoh D. et al., Proc. Natl. Acad. Sci. (U.S.A.) 1989, 86:1173; Gingeras T. R., et al., PCT Application WO 88/1D315), including nucleic acid sequence based amplification (NASBA) and 3SR. In NASBA, the nucleic acids can be prepared for amplification by standard phenol/chloroform extraction, heat denaturation of a clinical sample, treatment with lysis buffer and minispin columns for isolation of DNA and RNA or guanidinium chloride extraction of RNA. These amplification techniques involve annealing a primer which has ppGalNAc-T6 mRNA specific sequences. Following polymerization, DNA/RNA hybrids are digested with RNase H while double stranded DNA molecules are heat denatured again. In either case the single stranded DNA is made fully double stranded by addition of second tumor specific primer, followed by polymerization. The double stranded DNA molecules are then multiply transcribed by a polymerase such as T7 or SP6. In an isothermal cyclic reaction, the RNAs are reverse transcribed into double stranded DNA, and transcribed once against with a polymerase such as T7 or SP6. The resulting products, whether truncated or complete, indicate tumor specific sequences.

Davey C. et al., European Patent No. 0 329 822 disclose a nucleic acid amplification process involving cyclically synthesizing single-stranded RNA ("ssRNA"), ssDNA, and double-stranded DNA (dsDNA), which may be used in accordance with the present invention. The ssRNA is a first template for a first primer oligonucleotide, which is elongated by reverse transcriptase (RNA-dependent DNA polymerase). The RNA is then removed from resulting DNA: RNA duplex by the action of ribonuclease H (RNase H, an RNase specific for RNA in a duplex with either DNA or RNA). The resultant ssDNA is a second template for a second primer, which also includes the sequences of an RNA polymerase promoter (exemplified by T7 RNA polymerase) 5' to its homology to its template. This primer is then extended by DNA polymerase (exemplified by the large "Klenow" fragment of E. coli DNA polymerase I), resulting as a double-stranded DNA ("dsDNA") molecule, having a sequence identical to that of the original RNA between the primers and having additionally, at one end, a promoter sequence. This promoter sequence can be used by the appropriate RNA polymerase to make many RNA copies of the DNA. These copies can then reenter the cycle leading to very swift amplification. With proper choice of enzymes, this amplification can be done isothermally without addition of enzymes at each cycle.

Miller H. I. et al., PCT Application WO 89/06700 can be also cited which discloses a nucleic acid sequence amplification scheme based on the hybridization of a promoter/primer sequence to a target single-stranded DNA ("ssDNA") followed by transcription of many RNA copies of the sequence.

Methods based on ligation of two (or more) oligonucleotides in the presence of nucleic acid having the sequence of the resulting "di-oligonucleotide", thereby amplifying the di-oligonucleotide (Wu D. Y. et al., Genomics 1989,4: 560), may also be used in the amplification step of the present invention.

Following amplification, the presence or absence of the amplification product may be detected. The amplified product may be sequenced by any method known in the art. The sequenced amplified product is then compared to a sequence known to be in a prostate cancer specific sequence. Alternatively, the nucleic acids may be fragmented into varying sizes of discrete fragments. For example, DNA fragments may be separated according to molecular weight by methods such as and not limited to electrophoresis through an agarose gel matrix. The gels are then analyzed by Southern hybridization. Briefly, DNA in the gel is transferred to a hybridization substrate or matrix such as and not limited to a nitrocellulose sheet and a nylon membrane. A labelled probe is applied to the matrix under selected hybridization conditions so as to hybridize with complementary DNA localized on the matrix. The probe may be of a length capable of forming a stable duplex. The probe may have a size range from at least 15, 20, 25, 30, 35, 40, 45, 50, 60, 75, 100 or more nucleotides length to about 10,000 nucleotides, preferably about 100 to 200 nucleotides in length. Various labels for visualization or detection are known to those of skill in the art, such as and not limited to fluorescent staining, ethidium bromide staining for example, avidin/biotin, radioactive labelling such as ³²P labelling, and the like. Preferably, the product, such as the PCR product, may be run on an agarose gel and visualized using a stain such as ethidium bromide. The matrix may then be analyzed by autoradiography to locate particular fragments which hybridize to the probe.

In another embodiment, the invention relates to the methods according to the present invention, wherein the determining of the presence or the absence of ppGalNAc-T6 mRNA is carried out by a probe capable of specifically hybridizing with ppGalNAc-T6 mRNA, preferably hybridizing ppGalNAc-T6 mRNA having the sequence SEQ ID NO: 1, complementary sequence thereof or a fragment thereof having at least 15, 20, 25, 30, 35, 40, 45, 50, 60, 75, 100 or more nucleotides length which is specific of ppGalNAc-T6 mRNA, preferably human ppGalNAc-T6 mRNA.

A probe comprising at least a sequence which is complementary to the sequence SEQ ID NO: 3, or to a fragment thereof having at least 20, 25, 30, 35, 40, 45, 50, 60, 65 or 70 nucleotides length is more preferred.

In a particular embodiment, the invention relates to the methods according to the present invention, wherein the determining of the presence or the absence of ppGalNAc-T6 mRNA is carried out by the following method comprising the steps of:
(a) contacting a ppGalNAc-T6 nucleic acid probe under hybridizing conditions with the biological test sample comprising either;
   (i) RNA molecules isolated from a biological sample of the human subject, wherein the biological sample is suspected of containing tumor cells, or
   (ii) nucleic acid molecules synthesized from the isolated RNA molecules as cDNA;

   wherein the nucleic acid probe has a nucleotide sequence comprising either a fragment of at least 30 nucleotide length of the sequence of ppGalNAc-T6 mRNA having the sequence SEQ ID NO: 1, or its fragment having the sequence SEQ ID NO: 3, or their complement, and
(b) detecting the formation of hybrids of the nucleic acid probe and the test sample;

wherein the presence of hybrids indicates the presence of tumor cells in the tissue obtained from the human subject.

In embodiment of the above methods, the detecting of ppGalNAc-T6 RNA in a biological sample includes amplifying ppGalNAc-T6 mRNA. In another embodiment of the above methods, the detecting of ppGalNAc-T6 RNA in a biological sample includes hybridizing ppGalNAc-T6 mRNA with a probe.

One preferred method for the detection of mRNA levels involves contacting the isolated mRNA with a nucleic acid molecule (probe) that can hybridize to the mRNA encoded by the gene being detected. Probes based on the sequence of a nucleic acid molecule of the invention can be used to detect transcripts corresponding to ppGalNAc-T6 mRNA. The nucleic acid probe can be, for example, a full-length cDNA, or a fragment thereof, such as an oligonucleotide having a length sufficient to specifically hybridize under stringent conditions to ppGalNAc-T6 mRNA. Hybridization of an mRNA with the probe indicates that the marker in question is being expressed. In an embodiment, the probe includes a label group attached thereto, e. g., a radioisotope, a fluorescent compound, an enzyme, or an enzyme co-factor.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe (s), for example, in an Affymetrix gene chip array. A skilled artisan can readily adapt known mRNA detection methods for use in detecting the level of mRNA encoded by the markers of the present invention.

It is also an object of the invention to provide methods for diagnosing or prognosticating cancer in a subject, particularly for measuring the aggressiveness of cancer in a subject or for determining the presence of disseminated cancer cells in a cancer subject according to the present invention, wherein said gene ppGalNAc-T6 expression product is ppGalNAc-T6 peptide, preferably said ppGalNAc-T6 peptide having the sequence SEQ ID NO: 2.

When the methods according to the present invention are based to the detection or the quantification of ppGalNAc-T6 peptide expression, it is also preferred that the determination of the presence, or the level, or the absence of ppGalNAc-T6 by an immunohistochemical or an immunoassay method using an antibody capable of specifically recognizing said ppGalNAc-T6 peptide.

More preferred is an embodiment wherein said antibody is a monoclonal or polyclonal antibody directed against the ppGalNAc-T6 peptide fragment having the sequence SEQ ID NO: 4 (aa77-101 of the full length ppGalNAc-T6 peptide having the sequence SEQ ID NO: 2), or against an epitope fragment of at least 6, 7, 8, or 9 amino acids length of the peptide having the sequence SEQ ID NO: 4. Monoclonal antibodies are preferred.

In embodiments of the above methods, detection or assaying the level of ppGalNAc-T6 peptide in a biological sample from the subject includes contacting the biological sample with an antibody to ppGalNAc-T6 peptide or a fragment thereof; determining the binding (optionally its amount)of the antibody to the biological sample.

"Antibody" includes immunoglobulin molecules and immunologically active determinants of immunoglobulin molecules, i.e., molecules that contain an antigen binding site (epitope) which specifically binds (immunoreacts with) an antigen. Specificity of binding in the large and diverse set of antibodies is found in the variable (V) determinant of the H and L chains. Antibody includes polyclonal antibodies, monoclonal antibodies, whole immunoglobulins, and antigen binding fragments of the immunoglobulins.

The binding sites of the proteins that comprise an antibody, i.e., the antigen-binding functions of the antibody, are localized by analysis of fragments of a naturally occurring antibody. Thus, antigen-binding fragments are also intended to be designated by the term "antibody" Examples of binding fragments encompassed within the term antibody include: a Fab fragment consisting of the VL, VH, CL and Cm domains; an Fd fragment consisting of the VH and CHI domains; an Fv fragment consisting of the VL and VH domains of a single arm of an antibody; an isolated complementarity determining region (CDR); and an F(ab') 2 fragment, a bivalent fragment comprising two Fab' fragments linked by a disulfide bridge at the hinge region. These antibody fragments are obtained using conventional techniques well-known to those with skill in the art, and the fragments are screened for utility in the same manner as are intact antibodies. The term "antibody" is further intended to include bispecific and chimeric molecules having at least one antigen binding determinant derived from an antibody molecule.

In the diagnostic and prognostic assays of the invention, the antibody can be a polyclonal antibody or a monoclonal antibody and in a preferred embodiment is a labelled antibody.

Polyclonal antibodies are produced by immunizing animals, usually a mammal, by multiple subcutaneous or intraperitoneal injections of an immunogen (antigen) and an adjuvant as appropriate. As an illustrative embodiment, animals are typically immunized against a protein, peptide or derivative by combining about 1 ug to 1 mg of protein capable of eliciting an immune response, along with an enhancing carrier preparation, such as Freund's complete adjuvant, or an aggregating agent such as alum, and injecting the composition intradermally at multiple sites. Animals are later boosted with at least one subsequent administration of a lower amount, as 1/5 to 1/10 the original amount of immunogen in Freund's incomplete adjuvant (or other suitable adjuvant) by subcutaneous injection at multiple sites. Animals are subsequently bled, serum assayed to determine the specific antibody titer, and the animals are again boosted and assayed until the titer of antibody no longer increases (i.e., plateaus).

Such populations of antibody molecules are referred to as "polyclonal" because the population comprises a large set of antibodies each of which is specific for one of the many differing epitopes found in the immunogen, and each of which is characterized by a specific affinity for that epitope. An epitope is the smallest determinant of antigenicity, which for a protein may comprise a peptide of six to eight residues in length (Berzofsky J. and 1. Berkower (1993) in Paul, W., Ed., Fundamental Immunology, Raven Press, N. Y., p. 246). Affinities range from low, e. g. 10⁻⁶ M, to high, e. g., 10⁻¹¹.

The polyclonal antibody fraction collected from mammalian serum is isolated by well known techniques, e. g. by chromatography with an affinity matrix that selectively binds immunoglobulin molecules such as protein A, to obtain the IgG fraction. To enhance the purity and specificity of the antibody, the specific antibodies may be further purified by immunoaffinity chromatography using solid phase-affixed immunogen. The antibody is contacted with the solid phase-affixed immunogen for a period of time sufficient for the immunogen to immunoreact with the antibody molecules to form a solid phase-affixed immunocomplex. Bound antibodies are eluted from the solid phase by standard techniques, such as by the use of buffers of decreasing pH or increasing ionic strength, the eluted fractions are assayed, and those containing the specific antibodies are combined.

"Monoclonal antibody" as used herein refers to a preparation of antibody molecules of single molecular composition. A monoclonal antibody composition displays a single binding specificity and affinity for a particular epitope. Monoclonal antibodies can be prepared using a technique which provides for the production of antibody molecules by continuous growth of cells in culture. These include but are not limited to the hybridoma technique originally described by Kohler and Milstein (1975, Nature, 256: 495-497; see also Brown et al., 1981, J. Immunol., 127:539-46; Brown et al., 1980, J. Biol. Chem., 255:4980-83; Yeh et al., 1976, PNAS 76:2927-3I; and Yeh et al., 1982, Int. J. Cancer, 29:269-75) and the more recent human B cell hybridoma technique (Kozbor et al., 1983, Immunol. Today 4:72), EBV-hybridoma technique (Cole et al., 1985, Monoclonal Antibodies and Cancer Therapy, Alan R. Liss, Inc., pp. 77-96), and trioma techniques. The technology for producing hybridomas is well known (see generally Current Protocols in Immunology, Coligan et al. ed., John Wiley & Sons, New York, 1994). Hybridoma cells producing a monoclonal antibody of the invention are detected by screening the hybridoma culture supernatants for antibodies that bind the polypeptide of interest, e. g., using a standard ELISA assay.

A monoclonal antibody can be produced by the following steps. In all procedures, an animal is immunized with an antigen such as a protein (or peptide thereof) as described above for preparation of a polyclonal antibody. The immunization is typically accomplished by administering the immunogen to an immunologically competent mammal in an immunologically effective amount, i.e., an amount sufficient to produce an immune response. Preferably, the mammal is a rodent such as a rabbit, rat or mouse. The mammal is then maintained on a booster schedule for a time period sufficient for the mammal to generate high affinity antibody molecules as described. After a sufficient time to generate high affinity antibodies, the animal (e. g., mouse) is sacrificed and antibody-producing lymphocytes are obtained from one or more of the lymph nodes, spleens and peripheral blood. Spleen cells are preferred, and can be mechanically separated into individual cells in a physiological medium using methods well known to one of skill in the art. The antibody-producing cells are immortalized by fusion to cells of a mouse myeloma line.

Mouse lymphocytes give a high percentage of stable fusions with mouse homologous myelomas, however rat, rabbit and frog somatic cells can also be used. Spleen cells of the desired antibody-producing animals are immortalized by fusing with myeloma cells, generally in the presence of a fusing agent such as polyethylene glycol. Any of a number of myeloma cell lines suitable as a fusion partner can be used with to standard techniques, for example, the P3-NSI/1-Ag4-1, P3-x63-Ag8.653 or Sp2/O-Ag14 myeloma lines, available from the American Type Culture Collection (ATCC), Rockville, MD.

The fusion-product cells, which include the desired hybridomas, are cultured in selective medium such as HAT medium, designed to eliminate unfused parental myeloma or lymphocyte or spleen cells. Hybridoma cells are selected and are grown under limiting dilution conditions to obtain isolated clones. The supernatants of each clonal hybridoma is screened for production of antibody of desired specificity and affinity, e. g., by immunoassay techniques to determine the desired antigen such as that used for immunization. Monoclonal antibody is isolated from cultures of producing cells by conventional methods, such as ammonium sulfate precipitation, ion exchange chromatography, and affinity chromatography (Zola et al., Monoclonal Hybridoma Antibodies: Techniques And Applications, Hurell (ed.), pp. 51-52, CRC Press, 1982).

Hybridomas produced according to these methods can be propagated in culture *in vitro* or *in vivo* (in ascites fluid) using techniques well known to those with skill in the art.

Alternative to preparing monoclonal antibody-secreting hybridomas, a monoclonal antibody directed against a polypeptide of the invention can be identified and isolated by screening a recombinant combinatorial immunoglobulin library (e. g., an antibody phage display library) with the polypeptide of interest. Kits for generating and screening phage display libraries are commercially available (e. g., the Pharmacia Recombinant Phage Antibody System, Catalog No. 27-9400-01 ; and the Stratagene SutfZ4P Phage Display Kit, Catalog No. 240612). Additionally, examples of methods and reagents particularly amenable for use in generating and screening an antibody display library can be found in, for example, US Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al. (1991) Bio/Technology 9:1370-1372; Hay et al. (1992) Hum. Antibod. Hybridomas, 3:81-85; Huse et al. (1989) Science 246:1275-1281 ; Griffiths et al. (1993) EMBO J., 12: 725-734.

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in PCT Publication No. WO 87/02671; European Patent Application 0 184 187; European Patent Application 0 171 496; European Patent Application 0 173 494; PCT Publication No. WO 86/01533; U. S. Patent No. 4,816,567; European Patent Application 0 125 023; Better et al. (1988) "Labelled antibody" as used herein includes antibodies that are labeled by a detectable means and includes enzymatically, radioactively, fluorescently, chemiluminescently, and/or bioluminescently labeled antibodies by any of the many different methods known to those skilled in this art.

One of the ways in which an antibody can be detectably labelled is by linking the same to an enzyme. This enzyme, in turn, when later exposed to its substrate, will react with the substrate in such a manner as to produce a chemical moiety which can be detected, for example, by spectrophotometric, fluorometric or by visual means. Enzymes which can be used to detectably label a ppGalNAc-T6-specific antibody include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholinesterase.

Detection may be accomplished using any of a variety of immunoassays. For example, by radioactively labelling an antibody, it is possible to detect the antibody through the use of radioimmune assays. A description of a radioimmune assay (RIA) may be found in Laboratory Techniques and Biochemistry in Molecular Biology, by Work T. S. et al., North Holland Publishing Company, NY (1978), with particular reference to the chapter entitled "An Introduction to Radioimmune Assay and Related Techniques" by Chard T. The radioactive isotope can be detected by such means as the use of a gamma counter or a scintillation counter or by audioradiography. Isotopes which are particularly useful for the purpose of the present invention are: ³H, ¹³¹I, ³⁵S, 14C, and preferably ¹²⁵I.

It is also possible to label an antibody with a fluorescent compound. When the fluorescently labelled antibody is exposed to light of the proper wave length, its presence can then be detected due to fluorescence. Among the most commonly used fluorescent labelling compounds are fluorescein isothiocyanate, rhodamine, phycoerytherin, phycocyanin, allophycocyanin, ophthaldehyde and fluorescamine.

An antibody can also be detectably labelled using fluorescence emitting metals such as ¹⁵²Eu, or others of the lanthanide series. These metals can be attached to the antibody using such metal chelating groups as diethylenetriaminepentaacetic acid (DTPA) or ethylenediaminetetraacetic acid (EDTA).

An antibody can also be detectably labelled by coupling it to a chemiluminescent compound. The presence of the chemiluminescent-tagged antibody is then determined by detecting the presence of luminescence that arises during the course of a chemical reaction. Examples of particularly useful chemiluminescent labelling compounds are luminol, luciferin, isoluminol, theromatic acridinium ester, imidazole, acridinium salt and oxalate ester.

Likewise, a bioluminescent compound may be used to label an antibodyof the present invention. Bioluininescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labelling are luciferin, luciferase and aequorin.

In the detection assays of the invention, the amount of binding of the antibody to the biological sample can be determined by the intensity of the signal emitted by the labelled antibody and/or by the number cells in the biological sample bound to the labelled antibody.

The detection or the level of ppGalNAc-T6 in a biological sample may be determined by a radioimmunoassay, an immunoradiometric assay, and/or an enzyme immunoassay.

"Radioimmunoassay" is a technique for detecting and measuring the concentration of an antigen using a labelled (i.e. radioactively labelled) form of the antigen (i.e. ppGalNAc-T6 peptide). Examples of radioactive labels for antigens include ³H, ¹⁴C, and ¹²⁵I. The concentration of ppGalNAc-T6 in a biological sample is measured by having the antigen in the sample compete with a labelled (i.e. radioactively) antigen for binding to an antibody to the antigen. To ensure competitive binding between the labelled antigen and the unlabeled antigen, the labelled antigen is present in a sufficient concentration to saturate the binding sites of the antibody. The higher the concentration of antigen in the sample, the lower the concentration of labelled antigen that will bind to the antibody.

In a radioimmunoassay, to determine the concentration of labelled antigen bound to an antibody, the antigen-antibody complex must be separated from the free antigen. One method for separating the antigen-antibody complex from the free antigen is by precipitating the antigen-antibody complex with an anti-isotype antiserum. Another method for separating the antigen-antibody complex from the free antigen is by precipitating the antigen-antibody complex with formalin-killed *S*. *aureus.* Yet another method for separating the antigen-antibody complex from the free antigen is by performing a "solid-phase radioimmunoassay" where the antibody is linked (i.e. covalently) to Sepharose beads, polystyrene wells, polyvinylchloride wells, or microtiter wells. By comparing the concentration of labelled antigen bound to antibody to a standard curve based on samples having a known concentration of antigen, the concentration of antigen in the biological sample can be determined.

An "Immunoradiometric assay" (IRMA) is an immunoassay in which the antibody reagent is radioactively labeled. An IRMA requires the production of a multivalent antigen conjugate by techniques such as conjugation to a protein e.g., rabbit serum albumin (RSA). The multivalent antigen conjugate must have at least 2 antigen residues per molecule and the antigen residues must be of sufficient distance apart to allow binding by at least two antibodies to the antigen. For example, in an IRMA the multivalent antigen conjugate can be attached to a solid surface such as a plastic sphere.

Unlabeled "sample" antigen and antibody to antigen which is radioactively labelled are added to a test tube containing the multivalent antigen conjugate coated sphere. The antigen in the sample competes with the multivalent antigen conjugate for antigen antibody binding sites. After an appropriate incubation period, the unbound reactants are removed by washing and the amount of radioactivity on the solid phase is determined. The amount of bound radioactive antibody is inversely proportional to the concentration of antigen in the sample.

The most common enzyme immunoassay is the "Enzyme-Linked Immunosorbent Assay (ELISA)". The "Enzyme-Linked Immunosorbent Assay (ELISA)" is a technique for detecting and measuring the concentration of an antigen using a labelled (i.e. enzyme linked) form of the antibody.

In a "sandwich ELISA", an antibody (i.e. anti-ppGalNAc-T6 peptide) is linked to a solid phase (i.e. a microtiter plate) and exposed to a biological sample containing antigen (i.e. ppGalNAc-T6 peptide). The solid phase is then washed to remove unbound antigen. A labelled (i.e. enzyme linked) is then bound to the bound-antigen (if present) forming an antibody-antigen-antibody sandwich. Examples of enzymes that can be linked to the antibody are alkaline phosphatase, horseradish peroxidase, luciferase, urease, and 3-galactosidase. The enzyme linked antibody reacts with a substrate to generate a colored reaction product that can be assayed for.

In a "competitive ELISA", antibody is incubated with a sample containing antigen (i.e. ppGalNAc-T6 peptide). The antigen-antibody mixture is then contacted with an antigen-coated solid phase (i.e. a microtiter plate). The more antigen present in the sample, the less free antibody that will be available to bind to the solid phase. A labelled (i.e. enzyme linked) secondary antibody is then added to the solid phase to determine the amount of primary antibody bound to the solid phase.

In an "immunohistochemistry assay" a section of tissue is tested for specific proteins by exposing the tissue to antibodies that are specific for the protein that is being assayed. The antibodies are then visualized by any of a number of methods to determine the presence and amount of the protein present. Examples of methods used to visualize antibodies are, for example, through enzymes linked to the antibodies (e. g., luciferase, alkaline phosphatase, horseradish peroxidase, or P-galactosidase), or chemical methods (e.g., DAB/Substrate chromagen) or gold, fluorescent or labelled antibodies by any of the many different methods known to those skilled in this art.

In another aspect, the present invention concerns an *in vitro* method of screening for potential anti-cancer compounds, which comprises the steps of:
a) contacting the compound to be tested with a cell known to express ppGalNAc-T6 RNA, preferably mRNA, or ppGalNAc-T6 RNA; and
b) determining if such cells express ppGalNAc-T6 RNA or ppGalNAc-T6 peptide by a method of detecting ppGalNAc-T6 expression product as defined in claims 1 to 23, selecting said compound as a potential anti-cancer compounds if absence of ppGalNAc-T6 expression product is determined in the cells.

Preferably, the compounds to be tested are antisense RNA or interfering RNA (iRNA).

In another aspect, the present invention is directed to a method for the production of polyclonal antibodies capable of specifically recognizing ppGalNAc-T6 peptide, wherein such method comprises the step of:
a) immunization of a mammal animal with a immunologically effective amount of ppGalNAc-T6 peptide fragment having the sequence SEQ ID NO: 4, or against an epitope fragment of at least 9 amino acids length of the peptide having the sequence SEQ ID NO: 4, optionally with an enhancing carrier preparation;
b) optionally, *in vitro* determining the presence of specific antibodies in the animal serum or plasma; and
c) purifying or isolating the specific anti-ppGalNAc-T6 peptide from the animal serum or plasma.

It also forms part of the present invention a method for the production of an hybridoma cell capable of secreting monoclonal antibodies specifically recognizing ppGalNAc-T6 peptide, wherein such method comprises the step of:
a) immunization of a mammal animal with a immunologically effective amount of ppGalNAc-T6 peptide fragment having the sequence SEQ ID NO: 4, or against an epitope fragment of at least 9 amino acids length of the peptide having the sequence SEQ ID NO: 4, optionally with an enhancing carrier preparation;
b) isolating antibodies anti-ppGalNAc-T6 peptide producing lymphocytes from the spleen, lymph nodes or peripheral blood of that mammal animal; and
c) immortalizing the antibodies anti-ppGalNAc-T6 peptide producing lymphocytes by fusion of said lymphocytes to cells of same species mammal animal myeloma line.

It also forms part of the present invention a method for the production of monoclonal antibodies specifically recognizing ppGalNAc-T6 peptide, wherein such method comprises the step of:
a) producing an hybridoma cell capable of secreting monoclonal antibodies specifically recognizing ppGalNAc-T6 peptide according to the above method of the invention;
b) culturing such hybridoma cell in appropriate culture medium and culture conditions;
c) purifying or isolating from such culture medium the monoclonal antibodies which are secreted.

In another part, the present invention comprises polyclonal or monoclonal antibodies obtainable by the method for the production of polyclonal or monoclonal antibodies according to the present invention wherein such antibodies are specifically recognizing the ppGalNAc-T6 peptide fragment having the sequence SEQ ID NO: 4.

The hybridoma cells and the monoclonal antibody for the production of monoclonal antibodies which has been deposited at the Centre National de Collection de Microorganismes (C.N.C.M., Institut Pasteur, Paris, France) the 21 June 2004 under the accession number 1-3242.
and according to the Budapest Treaty, form part of the invention.

In another aspect, the present invention relates to kit for the detection or quantification of human ppGalNAc-T6 mRNA or peptide, wherein such kit comprises:
a) a polyclonal or monoclonal antibody according to the invention; or
b) a pair of primers selecting from the group consisting of a pair of primers capable of amplifying a fragment comprising the sequence SEQ ID NO: 3, or a fragment thereof having at least 30 nucleotides length which is specific of human ppGalNAc-T6 mRN, and the pair of primers having the sequence SEQ ID NO: 5 and SEQ ID NO: 6; or
c) a probe having a nucleotide sequence comprising either a fragment of at least 30 nucleotide length of the sequence of ppGalNAc-T6 mRNA having the sequence SEQ ID NO: 3, or their complement.

For antibody-based kits, the kit can comprise, for example: (1) a first antibody (e. g., in solution or attached a solid support) which binds to ppGalNAc-T6 peptide; and, optionally, (2) a second, different antibody conjugated to a detectable label which binds to either the ppGalNAc-T6 peptide or the first antibody.

For oligonucleotide-based kits, the kit can comprise, for example: (1) an oligonucleotide, e. g., a detectably labelled oligonucleotide, which hybridizes to ppGalNAc-T6 nucleic acid sequence (mRNA or cDNA, or specific fragment thereof) or (2) a pair of primers useful for amplifying a ppGalNAc-T6 nucleic acid molecule. The kit can also comprise, e. g., a buffering agent, a preservative, or a protein stabilizing agent. The kit can further comprise components necessary for detecting the detectable label (e. g., an enzyme or a substrate). The kit can also contain a control sample or a series of control samples which can be assayed and compared to the biological sample. Each component of the kit can be enclosed within an individual container and all of the various containers can be within a single package, along with instructions for interpreting the results of the assays performed using the kit.

The present invention also concerns a method for the complement of morphological diagnosis by the detection or the quantification of Human ppGalNAc-T6 gene product (mRNA or peptide) in biological sample of mammal, preferably a human by the above-cited methods or by a method well known by the skilled man, such immunohistochemical or immunoassay for the detection of the above defined peptide or by any method detecting the above defined nucleotide sequence (e.g. RT-PCR, quantitative RT-PCR, FISH, etc.) in cancer of any type (E;G; breast, colon, pancreas, head and neck, etc.).

The present invention also concerns a method for the evaluation of tumour aggressiveness and prognosis, in various types of cancer by the detection or the quantification of Human ppGalNAc-T6 gene product (mRNA or peptide) in biological sample of mammal, preferably a human by the above-cited methods or by a method well known by the skilled man, such immunohistochemical or immunoassay for the detection of the above defined peptide or by any method detecting the above defined nucleotide sequence (e.g. RT-PCR, quantitative RT-PCR, FISH, etc.) in cancer of any type (E;G; breast, colon, pancreas, head and neck, etc.).

The present invention also concerns a method for the detection of disseminated cancer cells (metastasis, micrometastasis, circulating tumour cells, etc.) in different tissues of cancer patients by the detection or the quantification of Human ppGalNAc-T6 gene product (mRNA or peptide) in biological sample of mammal, preferably a human by the above-cited methods or by a method well known by the skilled man, such immunohistochemical or immunoassay for the detection of the above defined peptide or by any method detecting the above defined nucleotide sequence (e.g. RT-PCR, quantitative RT-PCR, FISH, etc.).

The present invention also concerns a method for the indication and monitoring of treatment of cancer, in particular treatments targeted to the inhibition of ppGalNAc-T6 by the detection or the quantification of Human ppGalNAc-T6 gene product (mRNA or peptide) in biological sample of mammal, preferably a human by the above-cited methods or by a method well known by the skilled man, such immunohistochemical or immunoassay for the detection of the above defined peptide or by any method detecting the above defined nucleotide sequence (e.g. RT-PCR, quantitative RT-PCR, FISH, etc.) in cancer of any type (e.g. breast, colon, pancreas, head and neck).

Finally, when the methods according to the present invention are based to the detection or the quantification of ppGalNAc-T6 peptide expression, it is also preferred that the determination of the presence, or the level, or the absence of ppGalNAc-T6 by an immunohistochemical or an immunoassay method uses an antibody according to the present invention capable of specifically recognizing said ppGalNAc-T6 peptide.

The following examples and the figures are given for the purpose of illustrating various embodiments of the invention and are not meant to limit the present invention in any fashion.

### DESCRIPTION OF THE FIGURES

**Figure 1** shows representative results obtained using the procedures developed to co-amplify mNRA of ppGalNAc-T(1-9) and β-actin in appropriate positive control cell lines.
**Figure 2** shows the relationship between the disease free survival andmNRA expression in bone marrow samples from operable breast cancer patients, evaluated by RT-PCR of ppGalNAc-T6 (A), CEA (B) and CK19 (C).
**Figures 3A-3H** show the immunohistochemicalevaluation of anti-ppGalNAc-T6 monoclonal antibody on breast tissues. Antibodies react with the majority of breast cancer tissues, but did not with most normal breast tissues and non-malignant diseases evaluated.

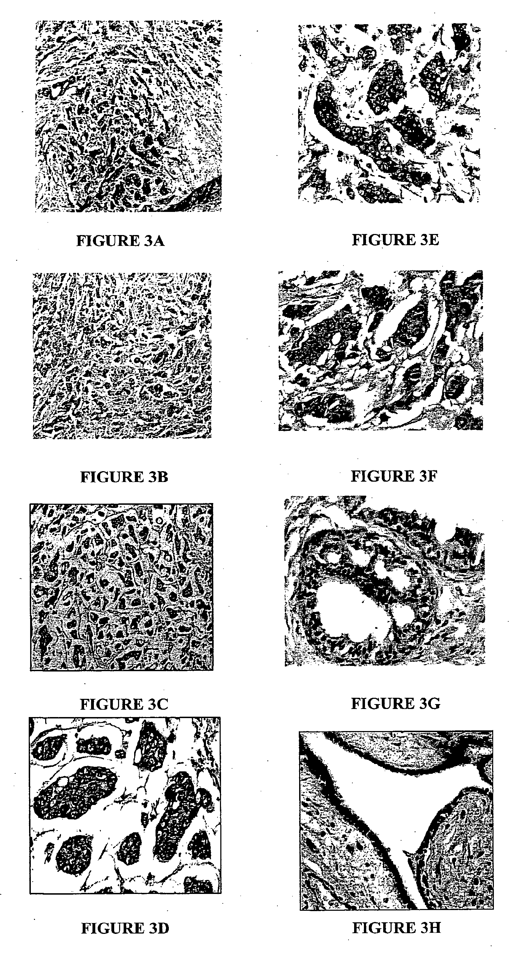

   Figure 3A: Ductal invasive breast cancer (100x): 80 % of malignant cells stained by monoclonal antibody anti-ppGalNAc-T6.
   Figure 3E: The same tumor as figure 3A (400x).
   Figure 3B: Ductal invasive breast cancer(100x). 70 % of malignant cells stained by monoclonal antibody anti-ppGalNAc-T6.
   Figure 3F: The same tumor as figure 3B (400x).
   Figure 3C: Ductal invasive breast cancer (100x). 95 % of malignant cells stained by monoclonal antibody anti-ppGalNAc-T6.
   Figure 3D: The same tumor as figure 3C (400x).
   Figure 3G: Moderate ductal hiperplasie(400x). Very weak focal staining by monoclonal antibody anti-ppGalNAc-T6.
   Figure 3H: Fibroadenome (400x). Non stained by monoclonal antibody anti-ppGalNAc-T6.

### EXAMPLES

First, it has been analysed the expression of 9 ppGalNAc-T genes (ppGalNAc-T1 to T9) in human breast cancer extracts and normal peripheral blood mononuclear (PBMN) cells using RT-PCR assays. The best results of sensitivity and specificity were obtained with the ppGalNAc-T6 assay. The inventors have thus assessed the expression of this gene in bone marrow aspirates obtained form 35 patients with operable breast cancer.

### Example 1:

### Patients and bone marrow aspirates

Bone marrow aspirates were obtained from 35 patients with histologic diagnosis of operable breast cancer at different stages. Our study was examined and approved by the ethical review board of Hospital de Clinicas, School of Medicine, Montevideo, Uruguay. An informed consent was obtained from all patients entered onto the protocol. All patients were screened for metastases by conventional staging (chest x-ray, liver ultrasound, blood tests and bone scan in patients with stage II or III). Five-milliliter samples of bone marrow aspirates were collected by sternal and iliac crest punctures, under anaesthesia before surgery, in EDTA anticoagulant and were quickly sent to the laboratory. Additionally, samples of peripheral blood cells were taken in the same conditions from healthy donors. Samples were first centrifuged at 1,500 g for 5 min and the buffy coats removed into fresh tubes. The contaminating red blood cells were eliminated by lysis buffer (154 mM ammonium chloride, 12 mM sodium bicarbonate and 0.1 mM EDTA) in gentle agitation for 10 min. The mononuclear cells were recovered by centrifugation at 1.500 g for 5 min., washed in PBS, recentrifuged and resuspended directly into Tri-Reagent (Sigma, St. Louis, MO). Tumour samples were pulverised in liquid nitrogen and then also resuspended in Tri-Reagent.

### Reverse transcriptase-polymerase chain reactions

Total RNA was extracted with Tri-Reagent according to the manufacturer's instructions from breast cancer cell lines, PBMN cells as well as from malignant breast tissues. First-strand cDNA was synthesised by using MMLV reverse transcriptase (Amersham, Piscataway, NJ) as reported previously (Berois et al., Anticancer Research, 17:2639-2646, 1997). Briefly, 4 µg of RNA was added to 200 units of enzyme in the presence of 2 µl of 20 mM of each deoxynucleotide triphosphate (dNTPs) and 1 µl 250 ng random hexamers in a total reaction volume of 20 µl. After incubation at 37°C for 1 hr, the mixture was heated to 96°C, snap-cooled and stored at -20°C. Different RT-PCR reactions, with the respective negative controls, were optimised in order to amplify fragments of ppGalNAc T1 to T9.

The sequence of primers utilised are shown in Table I.

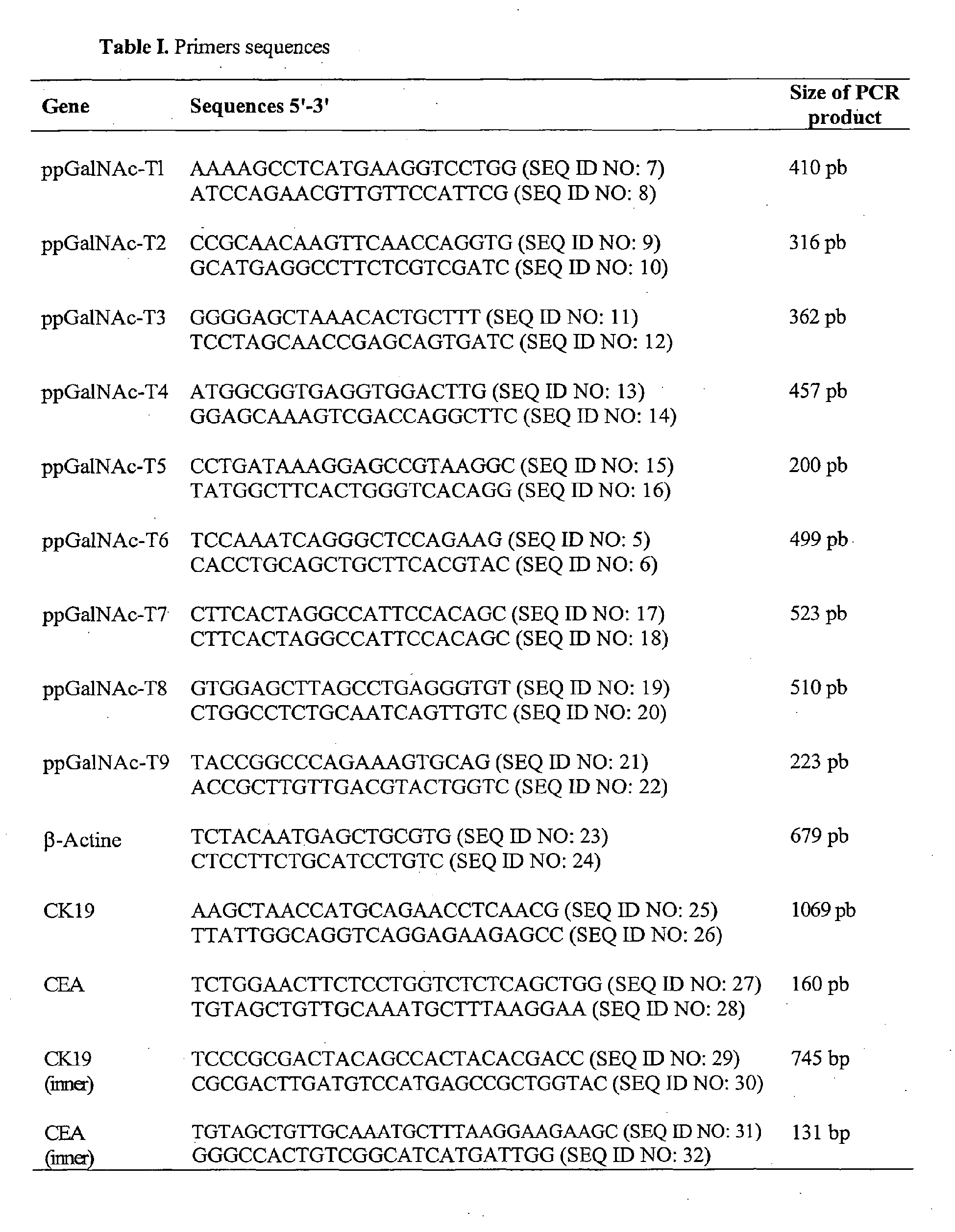

Amplification of the actine gene was carried out in the same PCR reaction tube in order to verify cDNA quality. 1 µl of cDNA was added to a final volume of 25 µl of a PCR mixture containing 20 mM Tris-HCl, pH 8.4, 50 mM KCl, 2.5 mM MgCl₂, 200 µM dNTPs, 300 nM each primer and 1 unit of Taq DNA polymerase (Invitrogen, Carlsbad, CA). Amplifications were performed under the following conditions for 35 cycles: 1 min. at 95°C, 1 min. at 57°C and 2 min. at 72°C. 15 µl of all PCR products were analysed by electrophoreses on 2 % agarose gels by direct visualisation after ethidium bromide staining.

### Example 2: ppGalNAc-Transferase gene expression in breast cancer tissues and normal PBMN cells.

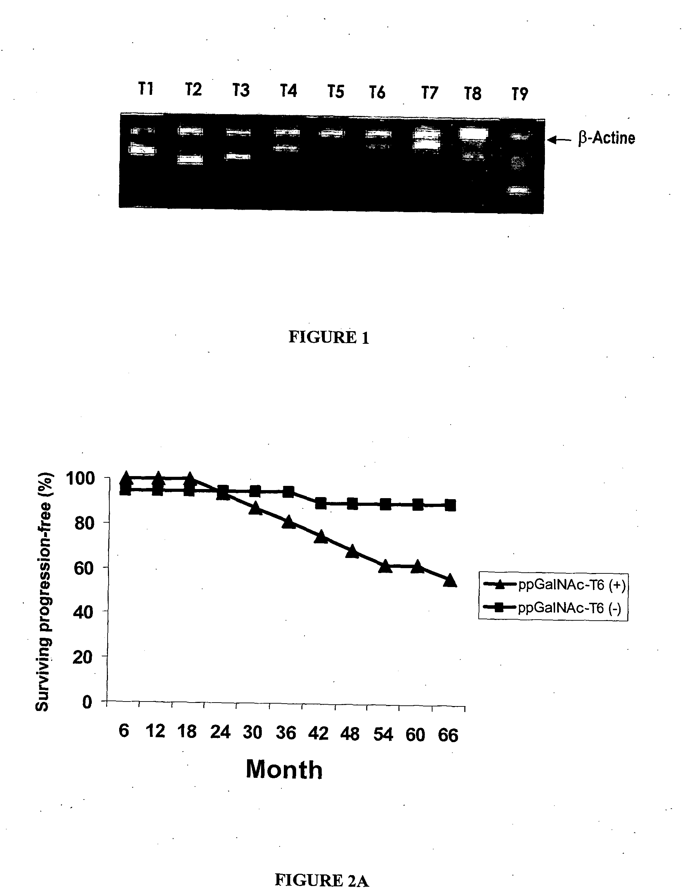

To evaluate the potential usefulness of ppGalNAc-T gene mRNA detection in the identification of disseminated breast cancer cells in bone marrow, the ppGalNAc-T(1-9) mRNA expression have been studied in breast cancer tissue samples and PBMN cells. Therefore, it has been developed an RT-PCR assay for each ppGalNAc-T from T1 to T9, which were co-amplified with a constitutive gene, the β-actine, which served as control to both RNA preparation and cDNA synthesis (figure 1). Although a group of ppGalNAc-Ts are found in breast cancer tissue samples (ppGalNAc-T1, -T2, -T3 and - T7), they are also found in-PBMN normal cells (see Table II).

**Table II. ppGalNAc-T expression in human breast tumours and PBMN cells**

| | **T1** | **T2** | **T3** | **T4** | **T5** | **T6** | **T7** | **T8** | **T9** |
|---|---|---|---|---|---|---|---|---|---|
| Breast tumors | 4/4 | 6/6 | 5/6 | 0/4 | 0/4 | 5/6 | 3/3 | 1/4 | 0/3 |
| PBMN cells | 4/4 | 6/6 | 3/3 | 0/3 | 0/6 | 0/5 | 2/4 | 1/4 | 0/3 |

Another group was not found neither in breast tumours, nor in PBMN control cells (ppGalNAc-T4, -T5, -T8 and -T9) or they were very little expressed (ppGalNAc-T8). Interestingly, ppGalNAc-T6 was found in the majority of studied breast cancer samples (5/6) and was absent in PBMN normal cells (0/5). Thus, this encouraged us to study the mRNA expression of this ppGalNAc-T in a bigger number of breast cancer samples and to evaluate their potential usefulness to detect disseminated breast cancer cells.

**Example 3:** ppGalNAc-T6 mRNA expression in human breast cancer cell lines and tissues ppGalNAc-T6 mRNA has been detected in the three human breast cancer cell lines evaluated (MCF-7, ZR75 and T47D) and in 22 of 25 human primary breast tumours (95 %), while the test was positive in only 1/30 (3 %) PBMN cell samples of healthy donors. Representative results of ppGalNAc-T6 RT-PCR assay for each mRNA sample are shown in Table II. To establish the limits of sensitivity of ppGalNAc-T6 RT-PCR assay for breast tumour cell detection, different number of T47D cells were evaluated. ppGalNAc-T6 was identified in tubes containing until 250 breast cancer T47D cells.

### Example 4: Analysis of bone marrow samples

ppGalNAc-T6 mRNA expression has been evaluated in bone marrow aspirates from 35 patients undergoing primary tumour resection. ppGalNAc-T6 was positive in 16 of 35 patients (40.5 %) (see Table III).

**Table III**

| Patient | Tumor size | Nodal involvement | Stage | pGalNAc-T6 | CK19 | CEA | Follow-up |
|---|---|---|---|---|---|---|---|
| 2 | 1 | NO | I | - | - | - | OK |
| 4 | 1 | YES | IIa | + | + | + | OK |
| 5 | 2 | NO | IIa | + | - | - | Relapse:28 |
| 6 | 2 | NO | IIa | + | - | - | OK |
| 9 | 2 | NO | IIa | - | - | - | OK |
| 11 | 2 | NO | IIa | + | + | + | OK |
| 12 | 1 | YES | IIa | + | + | + | OK |
| 14 | 1 | NO | I | - | + | - | OK |
| 19 | 2 | NO | IIa | + | + | + | Relapse: 62 |
| 20 | 2 | NO | IIa | - | + | + | OK |
| 21 | 1 | NO | I | - | - | - | OK |
| 22 | 2 | NO | IIa | + | - | - | OK |
| 25 | 2 | YES | Ilb | + | + | + | OK |
| 26 | 2 | YES | Ilb | + | + | + | Relapse: 39 |
| 27 | 2 | YES | Ilb | - | + | - | OK |
| 28 | 1 | YES | IIa | - | - | - | OK |
| 31 | 2 | YES | IIb | - | - | - | OK |
| 33 | 1 | NO | I | - | - | - | OK |
| 34 | 2 | NO | IIa | + | + | + | Relapse: 31 |
| 35 | 1 | NO | I | + | + | + | Relapse: 52 |
| 36 | 3 | YES | Illa | + | + | + | OK |
| 38 | 1 | YES | Ilb | - | - | - | OK |
| 40 | 2 | YES | Ilb | + | + | + | OK |
| 41 | 1 | NO | I | - | + | - | Relapse:6 |
| 42 | 1 | NO | I | - | + | + | OK |
| 44 | 1 | NO | I | - | - | - | OK |
| 50 | 1 | NO | I | + | - | - | Relapse: 44 |
| 52 | 3 | NO | Ilb | - | - | - | OK |
| 53 | 2 | YES | Ilb | - | - | - | OK |
| 60 | 2 | NO | IIa | - | + | - | OK |
| 63 | 2 | YES | I1b | - | + | - | OK |
| 64 | 2 | YES | I1b | + | + | + | Relapse:20 |
| 65 | 1 | NO | I | + | + | - | OK |
| 69 | 2 | YES | Ilb | - | - | - | Relapse:38 |
| 71 | 2 | YES | Ilb | - | + | - | OK |

Correlations between the ppGalNAc-T6 RT-PCR assay in bone marrow and various clinicopathologic parameters (stage, tumour size and nodal status) are shown in Table IV.

**Table IV: Correlations between the ppGalNAc-T6 RT-PCR assay in bone marrow and various clinicopathologic parameters**

| **TUMOUR SIZE** | | | | **LYMPH NODES** | | | **STAGE** | | |
|---|---|---|---|---|---|---|---|---|---|
| | **T1** | **T2** | **T3** | **NEG** | **POS** | **I** | **IIa** | **IIb** | **III** |
| **T6** | 5/14 | 10/19 | 1/2 | 9/20 | 7/15 | 3/10 | 8/12 | 4/12 | 1/1 |
| | (35.7%) | (53.6%) | (50%) | (45%) | (46.7%) | (30%) | (66.7%) | (33.3%) | (100%) |
| **CK19** | 7/14 | 12/19 | 1/2 | 10/20 | 10/15 | 5/10 | 7/12 | 7/12 | 1/1 |
| | (50%) | (63.6%) | (50%) | (50%) | (66.7%) | (50%) | (58.3%) | (58.3%) | (100%) |
| **CEA** | 4/14 | 8/19 | 1/2 | 6/20 | 7/15 | 2/10 | 6/12 | 4/12 | 1/1 |
| | (28.6%) | (42.1%) | (50%) | (30%) | (46.7%) | (20%) | (50%) | (33.3%) | (100%) |
| **ALL +** | 3/14 | 7/19 | 1/2 | 4/20 | 7/15 | 1/10 | 5/12 | 4/12 | 1/1 |
| | (21.4%) | (36.8%) | (50%) | (20%) | (46.7%) | (10%) | (41.2%) | (33.3%) | (100%) |

In all bone marrow samples, ppGalNAc-T6 mRNA expression was compared to RT-PCR assays for CEA and CK19, procedures extensively used to detect disseminated carcinoma cells (Table III). The study showed 20 of 35 patients (57.2 %) positive for CK19 mRNA and 13 of 35 patients (37.1 %) positive for CEA mRNA. It was observed that the three assays were negative in 10 of 35 patients (28.6 %). The three tests were positive in 11 of 35 patients (31.4 %). At least two procedures were positive in 14 of 35 patients (40 %). In 10 patients only one RT-PCR assay was observed (28.6 %), 6 patients for CK19 (17.1 %), 4 patients for ppGalNAc-T6 (11.4 %) and none for CEA (Table IV). In a follow-up evaluation of the 35 patients included in our study (median 6,1 years, minimum 5,1 years, maximum 7,2 years), 9 patients presented cancer relapse.

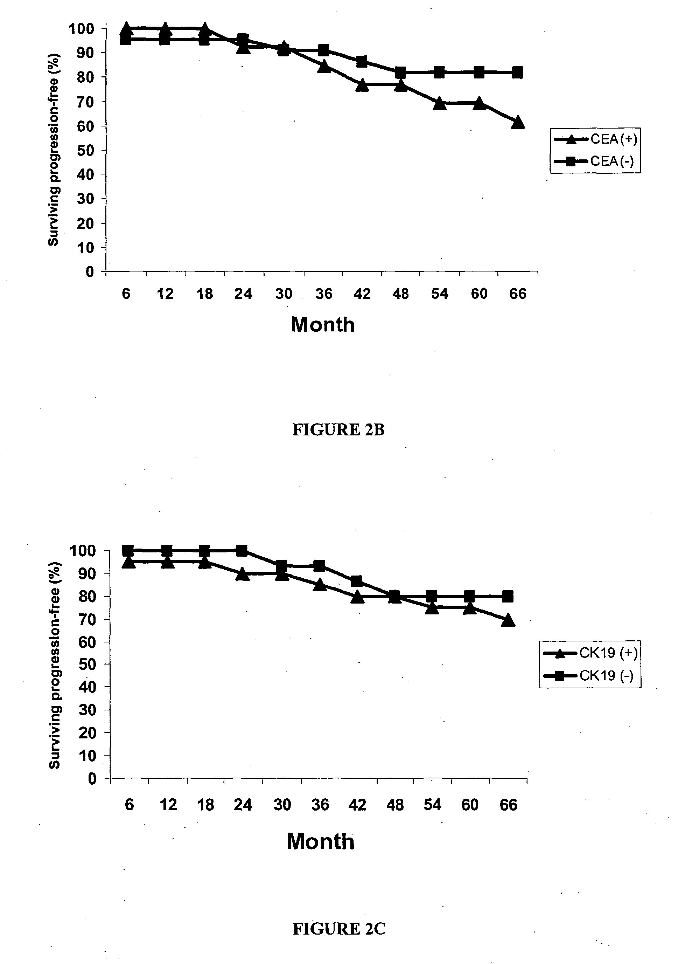

In this evaluation a positive ppGalNAc-T6 RT-PCR assay in bone marrow samples is associated with a high risk relapse of disease (see also figure 2). The results suggest that the ppGalNAc-T6 RT-PCR assay is a potential method applicable to the diagnosis of disseminated cells in breast cancer patients and that ppGalNAc-T6 is a new marker applicable to the diagnosis of disseminated cancer cells, particularly in breast cancer patients.

### Example 5: Production of monoclonal antibodies anti-ppGalNAc-T6 and characterisation of its reactivity in breast cancer tissues.

Monoclonal antibodies Anti-ppGalNAc-T6 have been produced using the synthetic peptide EAQQTLFSINQSCLPGFYTPAELKP (SEQ ID NO: 4) as immunogen have been produced. This amino acid sequence is within the segment amplified by RT-PCR with the pair of primers having the sequence SEQ ID NOs: 5 and 6. The amino acids coded by the fragment amplified by RT-PCR is the fragment aa71-235 of the sequence SEQ ID NO: 2. 14 hybridomas secreting antibodies specific against ppGalNAc-T6 have been established. Three of them were initially evaluated by immunohistochemistry on human breast tissues. These antibodies react with the majority of breast cancer tissues, but did not with most normal breast tissues and non-malignant diseases evaluated (Figure 3).

These antibodies are new tools useful for immunohistochemical or immunoassay diagnosis of cancer, preferably breast cancer.

Human ppGalNAc-T6 gene, located on chromosome 12q13, contains 10 exons of 1869 base pairs which encodes a type II trans-membrane protein. This gene, highly similar to the one which encodes ppGalNAc-T3, is expressed in a restricted pattern, being mainly in normal placenta, trachea, brain and pancreas. It has also been detected in a pancreatic adenocarcinoma cell line and in squamous carcinomas of the oral cavity (while it is not expressed in normal stratified squamous epithelium). Until present there is no available data about its expression in cancer, particularly in breast cancer.

## Claims

1. An *in vitro* method for diagnosing or prognosticating cancer in a subject, said method comprises the step of determining the presence or the absence of an expression product of the gene encoding ppGalNAc-T6 in a biological sample from the subject wherein the presence of ppGalNAc-T6 expression product is indicative of cancer.

2. An *in vitro* method for measuring the aggressiveness of cancer in a subject, said method comprises the step of determining the presence or the absence of the expression product of the gene encoding ppGalNAc-T6 in a biological sample from the subject wherein the presence of ppGalNAc-T6 expression product is indicative of the aggressiveness of the cancer.

3. An *in vitro* method for determining the presence of disseminated cancer cells in a biological sample from a subject with cancer, wherein said disseminated cancer cells are identifying by the presence of an expression product of the gene encoding ppGalNAc-T6 and wherein the presence of ppGalNAc-T6 expression product in cells is indicative of the presence of disseminated cancer cells.

4. An *in vitro* method for identifying metastatic cancer in a biological sample from a subject with a cancer or for determining if a subject is at risk of developing metastatic cancer, wherein said method comprises the step of determining the presence of disseminated cancer cells in said biological sample according to claim 3 and wherein the presence of disseminated cancer cells is indicative of metastatic cancer or is indicative of a cancer that will become metastatic.

5. An *in vitro* method for determining the subject's response to an anti-cancer therapy, in a subject who is receiving or has received therapy for a state associated with cancer wherein said method comprises the step of determining the presence or the absence of an expression product of the gene encoding ppGalNAc-T6 in a biological sample from the subject and the presence of the expression product of the gene encoding ppGalNAc-T6 is indicative of the subject's response to the therapy.

6. The method of one of claims 1 to 5, wherein the biological sample is selected from the group consisting of bone marrow, serum, plasma, blood, lymph, or cells from the cancerous or suspected cancerous tissue or adjacent tissue thereof.

7. The method according to one of claims 1 to 6, wherein said cancer is selected from the group consisting of breast cancer, ovarian cancer, digestive cancers and throat cancer.

8. The method according to claim 7, wherein said cancer is breast cancer.

9. The method according to claim 8, wherein said biological sample is a bone marrow sample.

10. The method according to one of claims 1 to 8, wherein said subject is a human subject.

11. The method according to one of claims 1 to 10, wherein said gene ppGalNAc-T6 expression product is ppGalNAc-T6 RNA, preferably mRNA.

12. The method according to claim 11, wherein said ppGalNAc-T6 mRNA has the sequence SEQ ID NO: 1.

13. The method according to claim 10 or 12, wherein the determining of the presence or the absence of ppGalNAc-T6 mRNA comprises a step of amplifying ppGalNAc-T6 mRNA or cDNA, the complementary sequence thereof, or a fragment thereof having at least 30 nucleotides length which is specific of ppGalNAc-T6, preferably said ppGalNAc-T6 mRNA having the sequence SEQ ID NO: 1.

14. The method according to claim 13, wherein the step of amplifying ppGalNAc-T6 mRNA or cDNA is carried out by PCR or RT-PCR reaction.

15. The method according to claim 14, wherein the pair of primers used for the PCR amplification is capable of amplifying a fragment comprising the sequence SEQ ID NO: 3 (nt212-711 of the cDNA having the sequence SEQ ID NO: 1 encoding ppGalNAc-T6), or a fragment thereof having at least 30 nucleotides length which is specific of human ppGalNAc-T6 mRNA.

16. A method according to claim 15, wherein said pair of primers has the sequence SEQ ID NO: 5 and SEQ ID NO: 6.

17. A method according to claim 11, wherein the determining of the presence or the absence of ppGalNAc-T6 mRNA is carried out by a probe capable of specifically hybridizing with ppGalNAc-T6 mRNA, preferably said ppGalNAc-T6 mRNA having the sequence SEQ ID NO: 1, complementary sequence thereof or a fragment thereof.

18. The method according to claim 17, wherein the probe comprises at least a sequence which is complementary to the sequence SEQ ID NO: 3, or to a fragment thereof having at least 30 nucleotides length which is specific ppGalNAc-T6 mRNA.

19. A method according to claim 17 or 18, comprising the steps of:
(a) contacting a ppGalNAc-T6 nucleic acid probe under hybridizing conditions with the biological test sample comprising either;
(i) RNA molecules isolated from a biological sample of the human subject, wherein the biological sample is suspected of containing tumor cells, or
(ii) nucleic acid molecules synthesized from the isolated RNA molecules as cDNA;
wherein the nucleic acid probe has a nucleotide sequence comprising either a fragment of at least 30 nucleotide length of the sequence of ppGalNAc-T6 mRNA having the sequence SEQ ID NO: 1, or its fragment having the sequence SEQ ID NO: 3, or their complement, and
(b) detecting the formation of hybrids of the nucleic acid probe and the test sample,
wherein the presence of hybrids indicates the presence of tumor cells in the tissue obtained from the human subject.

20. The method according to one of claims 1 to 10, wherein said gene ppGalNAc-T6 expression product is ppGalNAc-T6 peptide, preferably said ppGalNAc-T6 peptide having the sequence SEQ ID NO: 2.

21. A method according to claim 20, wherein the determining of the presence or the absence of ppGalNAc-T6 peptide is carried out by an immunohistochemical or an immunoassay method using an antibody capable of specifically recognizing said ppGalNAc-T6 peptide.

22. A method according to claim 21, wherein said antibody is a monoclonal or polyclonal antibody directed against the ppGalNAc-T6 peptide fragment having the sequence SEQ ID NO: 4 (aa77-101 of the full length ppGalNAc-T6 peptide having the sequence SEQ ID NO: 2), or against an epitope fragment of at least 9 amino acids length of the peptide having the sequence SEQ ID NO: 4.

23. The method of claim 22, wherein the antibody is a monoclonal antibody.

24. A method of screening for potential anti-cancer compounds, said method comprising the steps of:
a) contacting the compound to be tested with a cell known to express ppGalNAc-T6 RNA, preferably mRNA, or ppGalNAc-T6 RNA; and
b) determining if said cells express ppGalNAc-T6 RNA or ppGalNAc-T6 peptide by a method of detecting ppGalNAc-T6 expression product as defined in claims 1 to 23,
selecting said compound as a potential anti-cancer compounds if absence of ppGalNAc-T6 expression product is determined in the cells.

25. A method for the production of polyclonal antibodies capable of specifically recognizing ppGalNAc-T6 peptide, wherein said method comprises the step of:
a) immunization of a mammal animal with an immunologically effective amount of ppGalNAc-T6 peptide fragment having the sequence SEQ ID NO: 4, or against an epitope fragment of at least 9 amino acids length of the peptide having the sequence SEQ ID NO: 4, optionally with an enhancing carrier preparation;
b) optionally, *in vitro* determining the presence of specific antibodies in the animal serum or plasma; and
c) purifying or isolating from the animal serum or plasma the specific anti-ppGalNAc-T6 antibodies.

26. A method for the production of an hybridoma cell capable of secreting monoclonal antibodies specifically recognizing ppGalNAc-T6 peptide, wherein said method comprises the step of:
a) immunization of a mammal animal with an immunologically effective amount of ppGalNAc-T6 peptide fragment having the sequence SEQ ID NO: 4, or against an epitope fragment of at least 9 amino acids length of the peptide having the sequence SEQ ID NO: 4, optionally with an enhancing carrier preparation;
b) isolating antibodies anti-ppGalNAc-T6 peptide producing lymphocytes from the spleen, lymph nodes or peripheral blood of that mammal animal; and
c) immortalizing the antibodies anti-ppGalNAc-T6 peptide producing lymphocytes by fusion of said lymphocytes to cells of same species mammal animal myeloma line.

27. A method for the production of monoclonal antibodies specifically recognizing ppGalNAc-T6 peptide, wherein said method comprises the step of:
a) producing an hybridoma cell capable of secreting monoclonal antibodies specifically recognizing ppGalNAc-T6 peptide according to claim 26;
b) culturing said hybridoma cell in appropriate culture medium and culture conditions;
c) purifying or isolating from said culture medium the monoclonal antibodies which are secreted.

28. Polyclonal, or monoclonal antibodies obtainable by a method according to claims 25 and 27, wherein said antibodies are specifically recognizing the ppGalNAc-T6 peptide fragment having the sequence SEQ ID NO: 4.

29. The hybridoma cell according to claim 26, which has been deposited at the C.N.C.M (Institut Pasteur, Paris, France) the 21 June 2004 under the accession number I-3242.

30. Monoclonal antibody according to claim 27 or 28, which has been deposited at the C.N.C.M (Institut Pasteur, Paris, France) the 21 June 2004 under the accession number I-3242.

31. Kit for the detection or quantification of human ppGalNAc-T6 mRNA or peptide, wherein said kit comprises:
a) a polyclonal or monoclonal antibody according to claims28 and 30; or
b) a pair of primers selecting from the group consisting of pairs of primers capable of amplifying a fragment comprising the sequence SEQ ID NO: 3, or a fragment thereof having at least 30 nucleotides length which is specific of human ppGalNAc-T6 mRNA, and the pair of primers having the sequence SEQ ID NO: 5 and SEQ ID NO: 6; or
c) a probe having a nucleotide sequence comprising either a fragment of at least 30 nucleotide length of the sequence of ppGalNAc-T6 mRNA having the sequence SEQ ID NO: 3, or their complement.

32. The method according to claims 21 to 23, wherein the antibodies anti-ppGalNAc-T6 peptide is an antibody according to claims 28 and 30.


SEQUENCE LISTING

<110> INSTITUT CURIE

<120> GalNAc N-Acetylgalactosaminyl-Transferase 6 (ppGalNAc-T6) mRNA
      or peptide as a new marker for detection cancer cells

<130> D21838

<160> 32

<170> PatentIn version 3.2

<210> 1
<211> 2881
<212> DNA
<213> Homo sapiens

<220>
<223> Human cDNA coding for N-acetylgalactosaminyltransferase 6

<400> 1
atgaggctcc tccgcagacg ccacatgccc ctgcgcctgg ccatggtggg ctgcgccttt      60
gtgctcttcc tcttcctcct gcatagggat gtgagcagca gagaggaggc cacagagaag     120
ccgtggctga agtccctggt gagccggaag gatcacgtcc tggacctcat gctggaggcc     180
atgaacaacc ttagagattc aatgcccaag ctccaaatca gggctccaga agcccagcag     240
actctgttct ccataaacca gtcctgcctc cctgggttct ataccccagc tgaactgaag     300
cccttctggg aacggccacc acaggacccc aatgcccctg gggcagatgg aaaagcattt     360
cagaagagca agtggacccc cctggagacc caggaaaagg aagaaggcta taagaagcac     420
tgtttcaatg cctttgccag cgaccggatc tccctgcaga ggtccctggg gccagacacc     480
cgaccacctg agtgtgtgga ccagaagttc cggcgctgcc ccccactggc caccaccagc     540
gtgatcattg tgttccacaa cgaagcctgg tccacactgc tgcgaacagt gtacagcgtc     600
ctacacacca cccctgccat cttgctcaag gagatcatac tggtggatga tgccagcaca     660
gaggagcacc taaaggagaa gctggagcag tacgtgaagc agctgcaggt ggtgagggtg     720
gtgcggcagg aggagcggaa ggggttgatc accgcccggc tgctgggggc cagcgtggca     780
caggcggagg tgctcacgtt cctggatgcc cactgtgagt gcttccacgg ctggctggag     840
cccctcctgg ctcgaatcgc tgaggacaag acagtggtgg tgagcccaga catcgtcacc     900
atcgacctta atacttttga gttcgccaag cccgtccaga ggggcagagt ccatagccga     960
ggcaactttg actggagcct gaccttcggc tgggaaacac ttcctccaca tgagaagcag    1020
aggcgcaagg atgaaacata ccccatcaaa tccccgacgt ttgctggtgg cctcttctcc    1080
atccccaagt cctactttga gcacatcggt acctatgata atcagatgga gatctgggga    1140
ggggagaacg tggaaatgtc cttccgggtg tggcagtgtg ggggccagct ggagatcatc    1200
ccctgctctg tcgtaggcca tgtgttccgg accaagagcc cccacacctt ccccaagggc    1260
actagtgtca ttgctcgcaa tcaagtgcgc ctggcagagg tctggatgga cagctacaag    1320
aagattttct ataggagaaa tctgcaggca gcaaagatgg cccaagagaa atccttcggt    1380
gacatttcgg aacgactgca gctgagggaa caactgcact gtcacaactt ttcctggtac    1440
ctgcacaatg tctacccaga gatgtttgtt cctgacctga cgcccacctt ctatggtgcc    1500
atcaagaacc tcggcaccaa ccaatgcctg gatgtgggtg agaacaaccg cggggggaag    1560
cccctcatca tgtactcctg ccacggcctt ggcggcaacc agtactttga gtacacaact    1620
cagagggacc ttcgccacaa catcgcaaag cagctgtgtc tacatgtcag caagggtgct    1680
ctgggccttg ggagctgtca cttcactggc aagaatagcc aggtccccaa ggacgaggaa    1740
tgggaattgg cccaggatca gctcatcagg aactcaggat ctggtacctg cctgacatcc    1800
caggacaaaa agccagccat ggccccctgc aatcccagtg acccccatca gttgtggctc    1860
tttgtctagg acccagatca tccccagaga gagcccccac aagctcctca ggaaacagga    1920
ttgctgatgt ctgggaacct gatcaccagc ttctctggag gccgtaaaga tggatttcta    1980
aacccactgg gtggcaaggc aggaccttcc taatccttgc aacaacattg ggcccatttt    2040
ctttccttca caccgatgga agagaccatt aggacatata tttagcctag cgttttcctg    2100
ttctagaaat agaggctccc aaagtaggga aggcagctgg gggagggttc agggcagcaa    2160
tgctgagttc aagaaaagta cttcaggctg ggcacagtgg ctcatgcctg aaatcctagc    2220
actttgggaa gacaatgtgg gagaatggct tgagcccagg agttcaagac cggcctgagc    2280
aacatagtga ggatcccatc tctacgccca ccctcccccc ggcaaaaaaa aaagctgggt    2340

atggtggctt atgcctgtag tcgcagctac tcagaaggct gaggtgggag gattgcttgt 2400
tccccggagg ttgaagctac agtgagcctt gattgtgtca ctgcactcca gcctgggcaa 2460
caggtaagac tctgtctcaa aaaaaaaaca aaaaagaaga agaaaagtac ttctacagcc 2520
atgtcctatt ccttgatcat ccaaagcacc tgcagagtcc agtgaaatga tatattctgg 2580
ctgggcacag tggctcacac ctgtaatcct agcactttgg gaggccaagg caggtggatc 2640
acctgaggtc agaagtttga aaccagcctg gactacatgg tgaaactcca tctctactaa 2700
aagtacaaaa attagctggg catgatggca cgcacctgca gtcccagcta cttgggaggc 2760
tgaggcagga gaatcactcg aacccaggag gcagaggttg cagtgagcca agacagcacc 2820
attgcacccc agcctgagca acaagagcga aactccatct caggaaaaaa aaaaaaaaaa 2880
a 2881

<210> 2
<211> 622
<212> PRT
<213> Homo sapiens

<200>
<223> N-acetylgalactosaminyltransferase 6 polypeptide

<400> 2

Met Arg Leu Leu Arg Arg Arg His Met Pro Leu Arg Leu Ala Met Val
1               5                   10                  15

Gly Cys Ala Phe Val Leu Phe Leu Phe Leu Leu His Arg Asp Val Ser
            20                  25                  30

Ser Arg Glu Glu Ala Thr Glu Lys Pro Trp Leu Lys Ser Leu Val Ser
        35                  40                  45

Arg Lys Asp His Val Leu Asp Leu Met Leu Glu Ala Met Asn Asn Leu
    50                  55                  60

Arg Asp Ser Met Pro Lys Leu Gln Ile Arg Ala Pro Glu Ala Gln Gln
65                  70                  75                  80

Thr Leu Phe Ser Ile Asn Gln Ser Cys Leu Pro Gly Phe Tyr Thr Pro
                85                  90                  95

Ala Glu Leu Lys Pro Phe Trp Glu Arg Pro Pro Gln Asp Pro Asn Ala
            100                 105                 110

Pro Gly Ala Asp Gly Lys Ala Phe Gln Lys Ser Lys Trp Thr Pro Leu
        115                 120                 125

Glu Thr Gln Glu Lys Glu Glu Gly Tyr Lys Lys His Cys Phe Asn Ala
    130                 135                 140

Phe Ala Ser Asp Arg Ile Ser Leu Gln Arg Ser Leu Gly Pro Asp Thr
145                 150                 155                 160

Arg Pro Pro Glu Cys Val Asp Gln Lys Phe Arg Arg Cys Pro Pro Leu
                165                 170                 175

Ala Thr Thr Ser Val Ile Ile Val Phe His Asn Glu Ala Trp Ser Thr
            180                 185                 190

Leu Leu Arg Thr Val Tyr Ser Val Leu His Thr Thr Pro Ala Ile Leu
        195                 200                 205

Leu Lys Glu Ile Ile Leu Val Asp Asp Ala Ser Thr Glu Glu His Leu
    210                 215                 220

Lys Glu Lys Leu Glu Gln Tyr Val Lys Gln Leu Gln Val Val Arg Val
225                 230                 235                 240

Val Arg Gln Glu Glu Arg Lys Gly Leu Ile Thr Ala Arg Leu Leu Gly
                245                 250                 255

Ala Ser Val Ala Gln Ala Glu Val Leu Thr Phe Leu Asp Ala His Cys
            260                 265                 270

Glu Cys Phe His Gly Trp Leu Glu Pro Leu Leu Ala Arg Ile Ala Glu
        275                 280                 285

Asp Lys Thr Val Val Val Ser Pro Asp Ile Val Thr Ile Asp Leu Asn
    290                 295                 300

Thr Phe Glu Phe Ala Lys Pro Val Gln Arg Gly Arg Val His Ser Arg
305                 310                 315                 320

Gly Asn Phe Asp Trp Ser Leu Thr Phe Gly Trp Glu Thr Leu Pro Pro
                325                 330                 335

His Glu Lys Gln Arg Arg Lys Asp Glu Thr Tyr Pro Ile Lys Ser Pro
            340                 345                 350

Thr Phe Ala Gly Gly Leu Phe Ser Ile Pro Lys Ser Tyr Phe Glu His
        355                 360                 365

Ile Gly Thr Tyr Asp Asn Gln Met Glu Ile Trp Gly Gly Glu Asn Val
    370                 375                 380

Glu Met Ser Phe Arg Val Trp Gln Cys Gly Gly Gln Leu Glu Ile Ile
385                 390                 395                 400

Pro Cys Ser Val Val Gly His Val Phe Arg Thr Lys Ser Pro His Thr
                405                 410                 415

Phe Pro Lys Gly Thr Ser Val Ile Ala Arg Asn Gln Val Arg Leu Ala
            420                 425                 430

Glu Val Trp Met Asp Ser Tyr Lys Lys Ile Phe Tyr Arg Arg Asn Leu
        435                 440                 445

Gln Ala Ala Lys Met Ala Gln Glu Lys Ser Phe Gly Asp Ile Ser Glu
    450                 455                 460

Arg Leu Gln Leu Arg Glu Gln Leu His Cys His Asn Phe Ser Trp Tyr
465                 470                 475                 480

Leu His Asn Val Tyr Pro Glu Met Phe Val Pro Asp Leu Thr Pro Thr
                485                 490                 495

Phe Tyr Gly Ala Ile Lys Asn Leu Gly Thr Asn Gln Cys Leu Asp Val
            500                 505                 510

Gly Glu Asn Asn Arg Gly Gly Lys Pro Leu Ile Met Tyr Ser Cys His
        515                 520                 525

Gly Leu Gly Gly Asn Gln Tyr Phe Glu Tyr Thr Thr Gln Arg Asp Leu

530                 535                 540

Arg His Asn Ile Ala Lys Gln Leu Cys Leu His Val Ser Lys Gly Ala
545                 550                 555                 560

Leu Gly Leu Gly Ser Cys His Phe Thr Gly Lys Asn Ser Gln Val Pro
                565                 570                 575

Lys Asp Glu Glu Trp Glu Leu Ala Gln Asp Gln Leu Ile Arg Asn Ser
            580                 585                 590

Gly Ser Gly Thr Cys Leu Thr Ser Gln Asp Lys Lys Pro Ala Met Ala
        595                 600                 605

Pro Cys Asn Pro Ser Asp Pro His Gln Leu Trp Leu Phe Val
    610                 615                 620


<210> 3
<211> 500
<212> DNA
<213> Homo sapiens

<220>
<223> cDNA derived from Human cDNA coding for
      N-acetylgalactosaminyltransferase 6

<400> 3
tccaaatcag ggctccagaa gcccagcaga ctctgttctc cataaaccag tcctgcctcc      60
ctgggttcta taccccagct gaactgaagc ccttctggga acggccacca caggacccca     120
atgcccctgg ggcagatgga aaagcatttc agaagagcaa gtggaccccc ctggagaccc     180
aggaaaagga agaaggctat aagaagcact gtttcaatgc ctttgccagc gaccggatct     240
ccctgcagag gtccctgggg ccagacaccc gaccacctga gtgtgtggac cagaagttcc     300
ggcgctgccc cccactggcc accaccagcg tgatcattgt gttccacaac gaagcctggt     360
ccacactgct gcgaacagtg tacagcgtcc tacacaccac ccctgccatc ttgctcaagg     420
agatcatact ggtggatgat gccagcacag aggagcacct aaaggagaag ctggagcagt     480
acgtgaagca gctgcaggtg                                                 500


<210> 4
<211> 25
<212> PRT
<213> Homo sapiens

<220>
<223> peptide derived from Human for
      N-acetylgalactosaminyltransferase 6 polypeptide

<400> 4

Glu Ala Gln Gln Thr Leu Phe Ser Ile Asn Gln Ser Cys Leu Pro Gly
1               5                   10                  15

Phe Tyr Thr Pro Ala Glu Leu Lys Pro
            20                  25


<210> 5
<211> 21
<212> DNA
<213> Artificial

<220>
<223> Synthetic primer derived from human ppGalNAc-T6

<400> 5
tccaaatcag ggctccagaa g                                                21


<210> 6
<211> 22
<212> DNA
<213> Artificial

<220>
<223> Synthetic primer derived from human ppGalNAc-T6

<400> 6
cacctgcagc tgcttcacgt ac                                               22


<210> 7
<211> 23
<212> DNA
<213> Artificial

<220>
<223> Synthetic primer derived from human ppGalNAc-T1

<400> 7
aaaaagcctc atgaaggtcc tgg                                              23


<210> 8
<211> 22
<212> DNA
<213> Artificial

<220>
<223> Synthetic primer derived from human ppGalNAc-T1

<400> 8
atccagaacg ttgttccatt cg                                               22


<210> 9
<211> 22
<212> DNA
<213> Artificial

<220>
<223> Synthetic primer derived from human ppGalNAc-T2

<400> 9
ccgcaacaag ttcaaccagg tg                                               22


<210> 10
<211> 22
<212> DNA
<213> Artificial

<220>
<223> Synthetic primer derived from human ppGalNAc-T2

<400> 10
gcatgaggcc ttctcgtcga tc                                                22

<210> 11
<211> 20
<212> DNA
<213> Artificial

<220>
<223> Synthetic primer derived from human ppGalNAc-T3

<400> 11
ggggagctaa acactgcttt                                                   20

<210> 12
<211> 22
<212> DNA
<213> Artificial

<220>
<223> Synthetic primer derived from human ppGalNAc-T3

<400> 12
tcctagcaac cgagcagtga tc                                                22

<210> 13
<211> 20
<212> DNA
<213> Artificial

<220>
<223> Synthetic primer derived from human ppGalNAc-T4

<400> 13
atggcggtga ggtggacttg                                                   20

<210> 14
<211> 22
<212> DNA
<213> Artificial

<220>
<223> Synthetic primer derived from human ppGalNAc-T4

<400> 14
ggagcaaagt cgaccaggct tc                                                22

<210> 15
<211> 22
<212> DNA
<213> Artificial

<220>
<223> Synthetic primer derived from human ppGalNAc-T5

<400> 15
cctgataaag gagccgtaag gc                                                22

<210> 16
<211> 22
<212> DNA
<213> Artificial

<220>
<223> Synthetic primer derived from human ppGalNAc-T5

<400> 16
tatggcttca ctgggtcaca gg                                                22

<210> 17
<211> 22
<212> DNA
<213> Artificial

<220>
<223> Synthetic primer derived from human ppGalNAc-T7

<400> 17
cttcactagg ccattccaca gc                                                22

<210> 18
<211> 22
<212> DNA
<213> Artificial

<220>
<223> Synthetic primer derived from human ppGalNAc-T7

<400> 18
cttcactagg ccattccaca gc                                                22

<210> 19
<211> 22
<212> DNA
<213> Artificial

<220>
<223> Synthetic primer derived from human ppGalNAc-T8

<400> 19
gtggagctta gcctgagggt gt                                                22

<210> 20
<211> 22
<212> DNA
<213> Artificial

<220>
<223> Synthetic primer derived from human ppGalNAc-T8

<400> 20
ctggcctctg caatcagttg tc                                                22

<210> 21
<211> 20
<212> DNA
<213> Artificial

<220>
<223> Synthetic primer derived from human ppGalNAc-T9

<400> 21
taccggccca gaaagtgcag                                                   20

<210> 22
<211> 22
<212> DNA
<213> Artificial

<220>
<223> Synthetic primer derived from human ppGalNAc-T9

<400> 22
accgcttgtt gacgtactgg tc                                                22

<210> 23
<211> 18
<212> DNA
<213> Artificial

<220>
<223> Synthetic primer derived from human ß-actine

<400> 23
tctacaatga gctgcgtg                                                     18

<210> 24
<211> 18
<212> DNA
<213> Artificial

<220>
<223> Synthetic primer derived from human ß-actine

<400> 24
ctccttctgc atcctgtc                                                     18

<210> 25
<211> 25
<212> DNA
<213> Artificial

<220>
<223> Synthetic primer derived from human CK19

<400> 25
aagctaacca tgcagaacct caacg                                             25

<210> 26
<211> 25
<212> DNA
<213> Artificial

<220>
<223> Synthetic primer derived from human CK19

<400> 26
ttattggcag gtcaggagaa gagcc                                          25

<210> 27
<211> 29
<212> DNA
<213> Artificial

<220>
<223> Synthetic primer derived from human CEA

<400> 27
tctggaactt ctcctggtct ctcagctgg                                      29

<210> 28
<211> 27
<212> DNA
<213> Artificial

<220>
<223> Synthetic primer derived from human CEA

<400> 28
tgtagctgtt gcaaatgctt taaggaa                                        27

<210> 29
<211> 30
<212> DNA
<213> Artificial

<220>
<223> CK19 Inner primer

<400> 29
tcccgcgact acagccacta ctacacgacc                                     30

<210> 30
<211> 30
<212> DNA
<213> Artificial

<220>
<223> CK19 Inner primer

<400> 30
cgcgacttga tgtccatgag ccgctggtac                                     30

<210> 31
<211> 32
<212> DNA
<213> Artificial

<220>
<223> CEA Inner primer

<400> 31
tgtagctgtt gcaaatgctt taaggaagaa gc                                32

<210> 32
<211> 25
<212> DNA
<213> Artificial

<220>
<223> CEA Inner primer

<400> 32
gggccactgt cggcatcatg attgg                                        25